Europäisches Patentamt

European Patent Office

Office européen des brevets

(19)

(11) Publication number: **0 275 207 B1**

# EUROPEAN PATENT SPECIFICATION

(12)

(45) Date of publication of patent specification: **20.05.92**

(51) Int. Cl.⁵: **A61K 31/66**, A61K 7/32, A61K 7/06, A61K 7/40

(21) Application number: **88300327.9**

(22) Date of filing: **15.01.88**

(54) **Pharmaceutical and veterinary compositions.**

(30) Priority: **16.01.87 GB 8700942**
**26.05.87 GB 8712372**

(43) Date of publication of application:
**20.07.88 Bulletin 88/29**

(45) Publication of the grant of the patent:
**20.05.92 Bulletin 92/21**

(84) Designated Contracting States:
**AT BE CH DE ES FR GB GR IT LI LU NL SE**

(56) References cited:
**EP-A- 0 139 404**
**DE-A- 1 962 238**
**US-A- 3 892 845**
**US-A- 4 187 300**

**KATHON WT Manufacturers Instructions**

(73) Proprietor: **Albright & Wilson Limited**
**210-222 Hagley Road West**
**Oldbury Warley West Midlands B68 0NN(GB)**

(72) Inventor: **Talbot, Robert Eric**
**25 Sunfield Road**
**Cannock Staffordshire WS11 1NJ(GB)**
Inventor: **Davis, Keith Philip**
**3 Holly Close**
**Kinver Staffordshire DY7 6BP(GB)**
Inventor: **Smith, Brian**
**10 Foxhills Park**
**Netherton Dudley West Midlands DY2 0JO(GB)**

(74) Representative: **Savidge, Roger Gordon Madgwick et al**
**c/o Albright & Wilson Limited, P.O. Box 2098,7 Stars Road**
**Oldbury, Warley, West Midlands B69 4PR(GB)**

## Description

This invention relates to the pharmaceutical and veterinary compositions of hydroxyalkyl phosphines and their derivatives and especially to their use for topical application to humans and animals to cure, prevent or alleviate fungal, bacterial and other microbial infections of the skin, or to alleviate skin odour.

In our application E.P. 0139404 A1 we have described the use of certain hydroxyalkyl phosphines for water treatment and in our unpublished application GB 8527793 we have described horticultural and agricultural uses of hydroxyalkyl phosphines to control mosses lichens and plant pathogens. We have now discovered that hydroxyalkyl phosphines are effective against a wide range of fungal, bacterial, protozoal and other microbial infections of humans and animals.

Our invention provides a therapeutic or prophylactic composition comprising a hydroxyalkylphosphine compound of the formula $(HORPR^1{}_n)_xX_y$ wherein R is an alkylene group having from 1 to 4 carbon atoms and each $R^1$ may be the same or different and is an alkyl group having from 1 to 4 carbon atoms or an ROH group, X is an anion such that the compound is at least sparingly soluble in water, n is 2 or 3 and y is 0 or 1 such that $(n+y)$ is 2 or 4; or an at least sparingly water soluble condensate thereof; and a pharmaceutically or veterinarily acceptable carrier or diluent.

Preferably the hydroxyalkyl phosphine compound is tris (hydroxymethyl) phosphine or a precursor or most preferably a tetrakis (hydroxymethyl) phosphonium salt. Particularly preferred are tetrakis (hydroxymethyl) phosphonium chloride, sulphate, bromide and phosphate. However X may be any compatible anion such as nitrate, fluoride, a phosphonate such as acetodiphosphonate, aminotris-(methylenephosphonate), ethylenediamine tetrakis (methylenephosphonate) or diethylene triamine pentakis (methylene phosphonate), a condensed phosphate such as pyrophosphate, metaphosphate, tripolyphosphate or tetraphosphate, chlorate, chlorite, nitrite, sulphite, phosphite, hypophosphite, iodide, borate, metaborate, pyroborate, fluoborate or carbonate or an organic anion such as formate, acetate, benzoate, citrate, tartrate, lactate, propionate, butyrate, ethylene diamine tetracetate, paratoluene sulphonate, benzene sulphonate or a surfactant anion such as an alkyl benzene sulphonate, alkyl sulphate or alkyl ether sulphate.

The hydroxyalkyl phosphine compound may alternatively contain 2 or more phosphorus atoms, and preferably the phosphorus compound is water soluble to a concentration of at least 10 ppm. more preferably at least 20 ppm. most preferably at least 100 ppm e.g. at least 0.5 g/l at 25°C. Such phosphorus compounds contain at least 1 hydroxyalkyl group, usually per phosphorus atom, and preferably at least 2 hydroxyalkyl groups per phosphorus atom. Such hydroxyalkyl groups are preferably of formula ROH, where R is as defined above. The group or groups joining the phosphorus atoms together may be of formula -R-O, -R-O-R or -R-NH-R or -R-R"-R- where R is as defined above and R" is the residue formed by removal of two hydrogen atoms, bonded to nitrogen, from a di or polyamide or di or poly amine, such as urea, dicyandiamide, thiourea or guanidine. Such compounds with 2 or more, e.g. 3, hydroxyalkyl groups per phosphorus atom may be made by self condensation of compounds with 3 or 4 hydroxyalkyl groups attached to one phosphorus atom, e.g. of formula $[HOR\ P\ R'_nO_m]_yX_x$ or with a compound of the formula $R"H_2$ such as urea. Condensation may be performed by heating at 40-120°C.

The hydroxyalkyl phosphine compounds are highly effective against fungi such as Trichophyton rubrum, T. mentagrophytes, T. interdigitale and Microsporum canis which causes Athletes Foot, and/or Ringworm infections of humans.

The compounds are also very effective against various Streptococci such as S. pyogenes, which is responsible for a variety of human and animal diseases including Impetigo, Erysipalus, Scarlet Fever, Rheumatic Fever and Tonsilitis and other throat infections in humans and Mastitis of cattle, and S. Faecalis which is responsible for urinary infections such as Cystitis.

Other bacteria against which the compounds are effective include Proteus species such as P. mirabilis, P. vulgaris which cause urinary infections and pneumonia, Klebsiella pneumonia, which causes pneumonia, and womb infections of mares, salmonella typhimurium and S. Enteritides which cause food poisoning in humans and poultry, Shigella boydii which causes Bacillary Dysentery, Bacillus cereus which causes food poisoning and Legionella pneumophila which causes Legionaires Disease.

The compounds are also effective against Candida albicans and Candida tropicalis, which cause Thrush, Sporotrichum schenckii which causes abscessed in humans and horses, Aspergillus fumigatus which causes pulmonary disease and pathogenic protozoa such as Naegleria or Acanthamoeba which cause Amoebic Meningitis.

The composition according to the invention is applicable, for example, to the treatment of fungal infections such as Athletes Foot or Tinea (ringworm) and bacterial infections such as Boils, Erysipalus or Impetigo or to reduce foot order, or for the treatment of Mastitis in cattle.

2

The hydroxyalkyl phosphine compounds are especially useful in antiseptic/fungicidal creams, ointments, pastes, collodions, lotions, liniments and powders for treatment of Athletes Foot, Ringworm, Boils and other infections and inflammations of the skin, in foot sprays, and in veterinary dips and udder washes.

The compounds may also be used in mouth washes gargles for treating such condition as gum boils, Streptococcal infections and Oral Thrush. They are useful in pessaries and other preparations for treatment of Cystitis, Vaginal Thrush and other urinary and vaginal infections.

They may also be useful in topical, parenteral or oral preparations, for treatment, prevention and/or control of a variety of human and animal infections such as Salmonella infections in poultry and humans, Legionaires Disease, various types of Pneumonia, Staphylococcal infections, Rheumatic Fever, Plague, Scarlet Fever, Tuberculosis, Leprosy, Anthrax, protozoal infections, such as Amoebic Meningitis, Amoebic Dysentry, Malaria and Trypanosomiasis, vermal infections such as Hookworm, Filariasis, Toxocariasis, Bilharzia, Ascariasis, Tapeworm and Liver Fluke, and systemic fungal infections such as histoplasmosis, blastomycosis, paracoccidiomycosis, coccidiomycosis, and actinomycosis.

The composition may comprise an aqueous or organic solvent such as sterile water, saline solution, ethanol, isopropanol or glycerol, or mixtures thereof or a hydrocarbon, glyceride or other oil, preferably emulsified with water or an aqueous based solvent.

Particularly preferred are compositions adapted for topical application containing the hydroxyalkyl phosphine compound carried in a cream or ointment base or absorbed on or absorbed in an inert particulate solid such as talc, kaolinite, bentonite, diatomaceous earth, zeolite or other absorbent aluminosilicate or calcium carbonate. Alternatively the compounds may be encapsulated or micro capsulated in a pharmaceutically acceptable encapsulant such as sugar, fat, gelatin, resin or gum, such as gum acacia, gum arabic, gum tragacanth or a slowly soluble synthetic polymer, such as polyvinylalcohol or absorbed in a slow release of glass such as a phosphate glass.

Alternatively the compounds or solutions thereof may respectively be dissolved in or emulsified with liquified gaseous solvents in pressurised containers. The invention also provides medicated shampoos and medicated soap in bar, liquid crystal or liquid form containing the hydroxyalkyl phosphine compounds.

The compounds may be tabletted with suitable solids such as chalk, kaolin, sugar or salt or dissolved in syrups, linctus or saline drips.

The compositions may additionally contain other fungicides, bactericides or synergists, antiperspirants, lanolin or other skin softening or moisturising preparations, analgesics, antibiotics, emulsifiers, dispersants, carboxymethyl cellulose, soaps, surfactants, polymeric thickening agents, wetting agents, foam controlling agents, perfumes, flavourings, colouring, deodorants, reodorants and antiseptics.

A typical ointment, for instance, may contain soft paraffin, petroleum jelly, emulsifying wax, and/or wool fat, optionally in admixture with liquid paraffin and/or hard paraffin, a cream may typically comprise an aqueous based emulsion of emulsifying wax, together optionally with soft paraffin or other hydrocarbons, and/or wool fat. Creams may optionally contain buffers, such as citric acid/sodium phosphate. Ointments or creams may be combined with e.g. zinc oxide or calamine to form pastes.

The effective dose depends upon the nature and site of the infection. Typically the hydroxyalkylphosphine compounds exhibit bacterial and fungicidal activity at concentrations within the range 1 to 2000 ppm, especially 5 to 1000, preferably 10 to 500 e.g. 15 to 200 ppm. It is preferred to administer doses adapted to provide such concentrations at the site of infection, subject to toxicological constraints.

Compositions according to the invention typically contain from 0.2 to 20% by weight of the hydroxyalkylphosphine compound preferably 0.5 to 10% especially 1 to 3% by weight. Aqueous solutions up to 80% by weight concentration are obtainable, but these normally require some dilution to provide compositions suitable to be administered to humans or animals. Our invention includes concentrates for dilution to provide pharmaceutical, veterinary or cosmetic preparations. THP salts are generally non-carcinogenic and non-mutagenic, but exhibit toxic effects especially on liver cells. Their toxicity in topical applications is generally low, but care must be taken in administering them internally.

The invention is of particular value for the treatment of vertebrates including: mammals such as cats, dogs and other carnivores, horses, sheep, pigs, cattle, goats, camels, deer and other ungulates, mice, hamsters, gerbils and other rodents, man and other primates; birds such as chickens, geese, ducks, turkeys and cage birds; reptiles; and fish.

The invention will be illustrated by the following examples.

Example 1

In the following Tables 2 to 21 the material D denotes a 75% by weight aqueous solution of tetrakis-(hydroxymethyl) phosphonium sulphate and Kathon WT® is a registered Trade Mark and represents a widely used proprietory biocide which is an aqueous solution of a mixture of 2-methyl-4-isothiazolin-3-one and 5-chloro-2-methyl-4-isothiazolin-3-one. The results show that material D has a high degree of lethal activity to bacteria within 2 to 6 hours of exposure at concentrations of at least 20 ppm and a high degree of lethal activity to the fungi at 100-150 ppm after 24 hours.

**TABLE 1**

(a) Bacteria

The following bacterial strains were used in the tests:
Streptococcus pyogenes NCTC 8198
Streptococcus faecalis ATCC 19433
Sarcina lutea ATCC 9341
Proteus mirabilis NCTC 8559
Proteus vulgaris NCTB 4175
Klebsiella pneumoniae NCTC 10341
Salmonella typhimurium NCTB 10258
Salmonella enteritidis NCTC 6676
Shigella boydii NCTC 9328
Bacillus subtilus ATCC 6633
Bacillus cereus ATCC 11778

(b) Yeasts and fungi

The following yeast and fungal strains were used in the tests:
Candida albicans ATCC 10231
Candida tropicalis NCPF 3111
Absidia corymbifera NCPF 2001
Sporotrichum schenkii NCPF 3182
Trichophyton rubrum NCPF 197
Trichophyton mentagrophytes NCPF 351
Microsporum Canis NCPF 351
Penicillium funicolosum CMI 114933
Aureobasidium pullulans CMI 45533

Inocula for the bacterial strains were prepared from cultures on Tryptone soy agar (TSA, Oxoid CM 131) incubated at 37°C for 24 hours. Surface growth of the organisms was suspended in sterile physiological saline and diluted with this medium to give 50% transmission at 520 nm on an SP 600 spectrophotometer, an approximate yield of $10^8$ colony forming units (cfu) per ml. For use as inocula these suspensions were further diluted in sterile physiological saline to give an expected fnal concentration in the test solutions of $10^4$ to $10^5$ of cfu/ml.

Inocula for the two yeast (Candida) strains wre prepared from cultures on Sabouraud dextrose agar (SDA Oxide CM 141) incubated at 37°C for 48 hours. Surface growth of the organisms was suspended in sterile physiological saline and standardised by plate counts to contain approximatedly $10^6$ cfu/ml.

For use as inocula these suspensions were diluted to give an expected final concentration in the test solutions of $10^4$ to $10^5$ cfu/ml.

Inocula for the fungal strains were prepared by sub-culturing the organisms from SDA into Sabouraud liquid medium (SLM, Oxoid 147) and incubating at 30°C for 5 to 10 days. Mycelial growth was then broken down by shaking the cultures with glass beads and the resulting suspensions were standardised by plated counts to contain approximately $10^6$ cfu/ml.

All tests were carried out in phosphate buffered saline (PBS) prepared from Oxoid tablets (Dulbecco A, Oxoid BR14a) and having the formula:
Sodium chloride 8 g/litre
Potassium chloride 0.2 g/litre

4

Disodium hydrogen phosphate 115 g/litre
Potassium dihydrogen phosphate 0.2 g/litre
pH 7.3 g/litre

Samples from the inoculated test and control preparations were diluted 1 ml to 9 ml in 0.1% peptons water containing 1% v/v Tween® 80 as an inactivator, and serially diluted in further ten-fold steps in the same dilutent.

The media used for preparing plate counts from the diluted samples were:

(a) for bacterial strains - Tryptone soy agar containing 0.2% sodium thioglycollate

(b) for yeast and fungal strains - Sabouraud dextrose agar containing 0.2% sodium thioglycollate.

For preparation of the test series each was diluted in PBS 1 part in 1000 parts to give a stock solution of 1000 ppm. This was freshly prepared on each test day.

The stock dilution was then further diluted to give the test series of:

(b) 500, 200, 100, 50 and 20 ppm for the organisms tested Strep. pyogenes, Strep. faecalis, Shigella boydii, Salmonella enteritidis and Klebsiella pneumoniae) and:

(c) 500, 100, 20, 5 and 1 ppm for the remainder of the test organisms.

The test solutions as described in (5) above were prepared in 50 ml volumes and the test solutions plus a 100 ml volume of PBS (as control) were inoculated with appropriate volumes (0.5 to 1 ml) of the inocula prepared as described in (2) above, shaken to evenly distribute the inoculum and held at room temperature. Immediatedly after inoculation (time 0) and then at pre-determined times 0.1 ml aliquots were removed from the test and control solutions for counting.

The 0.1 ml samples were serially diluted in hundred-fold steps in the peptons water/Tween® 80 diluent and 1 ml volumes of the dilutions were used to prepare duplicate pour-plates in TSA/sodium thioglycollate medium for the bacteria and Sabouraud/sodium thioglycollate medium for the yeasts and fungi.

The bacterial count plates were incubated at 37°C for 24 hours, the yeast (Candida) count plates at 37°C for 48 hours and the fungal count plates at 30°C for 5 to 10 days.

Developing colonies were then counted and by reference to the appropriate dilution ratios the numbers of surviving organisms at each compound concentration/time point were calculated.

## TABLE 2

Recovery of viable cells of Streptococcus pyogenes NCTC 8198
following exposure to D and Kathon® WT in
phosphate buffered saline

| Compound<br><br>Conc. in ppm | Recovered viable cells cfu/ml | | | |
|---|---|---|---|---|
| | Sampling time after inoculation | | | |
| | 0 | 2 h | 6 h | 24 h |
| Material D | | | | |
| 500 ppm | $1.8 \times 10^5$ | 0 | 0 | 0 |
| 200 ppm | $1.2 \times 10^5$ | 0 | 0 | 0 |
| 100 ppm | $1.4 \times 10^5$ | 0 | 0 | 0 |
| 50 ppm | $1.4 \times 10^5$ | 0 | 0 | 0 |
| 20 ppm | $2.0 \times 10^5$ | $1.7 \times 10^2$ | 0 | 0 |
| Kathon® WT | | | | |
| 500 ppm | $2.8 \times 10^5$ | $4.4 \times 10^5$ | $3.0 \times 10^5$ | $1.1 \times 10^5$ |
| 200 ppm | $8.0 \times 10^4$ | $2.1 \times 10^5$ | $2.8 \times 10^5$ | $1.2 \times 10^5$ |
| 100 ppm | $1.4 \times 10^5$ | $3.6 \times 10^5$ | $1.9 \times 10^5$ | $3.0 \times 10^5$ |
| 50 ppm | $2.1 \times 10^5$ | $7.2 \times 10^5$ | $2.3 \times 10^5$ | $2.2 \times 10^5$ |
| 20 ppm | $1.4 \times 10^5$ | $6.0 \times 10^5$ | $2.2 \times 10^5$ | $6.0 \times 10^5$ |
| Control<br>(phosphate buffered<br>saline) | $8.7 \times 10^5$ | $3.4 \times 10^5$ | $1.8 \times 10^5$ | $3.7 \times 10^5$ |

## TABLE 3

Recovery of viable cells of Streptococcus faecalis ATCC 19433 following exposure to D and Kathon® WT in phosphate buffered saline

| Compound | Recovered viable cells cfu/ml | | | |
|---|---|---|---|---|
| Conc. in ppm | Sampling time after inoculation | | | |
| | 0 | 2 h | 6 h | 24 h |
| Material D | | | | |
| 500 ppm | $1.3 \times 10^5$ | 0 | 0 | 0 |
| 200 ppm | $3.2 \times 10^5$ | 0 | 0 | 0 |
| 100 ppm | $2.8 \times 10^5$ | 0 | 0 | 0 |
| 50 ppm | $3.0 \times 10^5$ | 0 | 0 | 0 |
| 20 ppm | $4.1 \times 10^5$ | 20 | 0 | 0 |
| Kathon® WT | | | | |
| 500 ppm | $2.6 \times 10^5$ | $1.5 \times 10^5$ | $1.2 \times 10^5$ | $1.4 \times 10^5$ |
| 200 ppm | $6.5 \times 10^5$ | $1.6 \times 10^5$ | $1.9 \times 10^5$ | $1.2 \times 10^5$ |
| 100 ppm | $5.8 \times 10^5$ | $1.4 \times 10^5$ | $1.2 \times 10^5$ | $5.0 \times 10^4$ |
| 50 ppm | $3.3 \times 10^5$ | $1.9 \times 10^5$ | $1.9 \times 10^5$ | $1.8 \times 10^4$ |
| 20 ppm | $3.8 \times 10^5$. | $2.6 \times 10^5$ | $1.1 \times 10^5$ | $2.7 \times 10^4$ |
| Control (phosphate buffered saline) | $3.8 \times 10^5$ | $2.0 \times 10^5$ | $1.6 \times 10^5$ | $3.1 \times 10^4$ |

## TABLE 4

Recovery of viable cells of Sarcina lutea ATC 9341
following exposure to D and Kathon® WT in
phosphate buffered saline

| Compound<br><br>Conc. in ppm | Recovered viable cells cfu/ml | | | |
|---|---|---|---|---|
| | Sampling time after inoculation | | | |
| | 0 | 2 h | 6 h | 24 h |
| Material D | | | | |
| 500 ppm | $1.4 \times 10^4$ | 0 | 0 | 0 |
| 200 ppm | $1.7 \times 10^4$ | 0 | 0 | 0 |
| 100 ppm | $1.5 \times 10^4$ | $1.6 \times 10^4$ | $5.5 \times 10^3$ | 60 |
| 50 ppm | $1.6 \times 10^4$ | $1.2 \times 10^4$ | $1.8 \times 10^4$ | $6.9 \times 10^5$ |
| 20 ppm | $1.4 \times 10^4$ | $1.6 \times 10^4$ | $1.8 \times 10^4$ | $5.6 \times 10^5$ |
| Kathon® WT | | | | |
| 500 ppm | $1.4 \times 10^4$ | $1.3 \times 10^4$ | $1.4 \times 10^4$ | $1.7 \times 10^4$ |
| 200 ppm | $1.6 \times 10^4$ | $1.5 \times 10^4$ | $1.5 \times 10^4$ | $2.0 \times 10^4$ |
| 100 ppm | $1.7 \times 10^4$ | $1.8 \times 10^4$ | $1.8 \times 10^4$ | $1.8 \times 10^4$ |
| 50 ppm | $1.8 \times 10^4$ | $1.6 \times 10^4$ | $1.6 \times 10^4$ | $1.8 \times 10^4$ |
| 20 ppm | $1.5 \times 10^4$ | $1.6 \times 10^4$ | $5.0 \times 10^4$ | $2.2 \times 10^4$ |
| Control<br>(phosphate buffered<br>saline) | $1.4 \times 10^4$ | $2.0 \times 10^4$ | $1.6 \times 10^4$ | $1.4 \times 10^6$ |

## TABLE 5

Recovery of viable cells of Proteus mirabilis NCTC 8559 following exposure to D and Kathon® WT in phosphate buffered saline

| Compound Conc. in ppm | Recovered viable cells cfu/ml Sampling time after inoculation | | | |
|---|---|---|---|---|
| | 0 | 2 h | 6 h | 24 h |
| **Material D** | | | | |
| 500 ppm | $1.0 \times 10^5$ | 0 | 0 | 0 |
| 100 ppm | $9.5 \times 10^5$ | 0 | 0 | 0 |
| 20 ppm | $1.0 \times 10^6$ | 0 | 0 | 0 |
| 5 ppm | $1.3 \times 10^6$ | $1.2 \times 10^6$ | $1.5 \times 10^6$ | $4.1 \times 10^6$ |
| 1 ppm | $1.6 \times 10^6$ | $1.2 \times 10^6$ | $2.8 \times 10^6$ | $9.0 \times 10^6$ |
| **Kathon WT®** | | | | |
| 500 ppm | $1.0 \times 10^6$ | $1.7 \times 10^5$ | $4.0 \times 10^2$ | 0 |
| 100 ppm | $1.2 \times 10^6$ | $1.3 \times 10^5$ | $1.9 \times 10^3$ | 0 |
| 20 ppm | $1.1 \times 10^6$ | $5.3 \times 10^5$ | $2.5 \times 10^5$ | $5.0 \times 10^2$ |
| 5 ppm | $1.2 \times 10^6$ | $6.5 \times 10^5$ | $2.0 \times 10^5$ | $1.7 \times 10^2$ |
| 1 ppm | $1.2 \times 10^6$ | $9.7 \times 10^5$ | $2.9 \times 10^5$ | $1.7 \times 10^2$ |
| Control (phosphate buffered saline) | $1.2 \times 10^6$ | $1.0 \times 10^6$ | $2.3 \times 10^6$ | $9.8 \times 10^6$ |

## TABLE 6

Recovery of viable cells of Proteus vulgaris NCIB 4175
following exposure to D and Kathon® WT in
phosphate buffered saline

| Compound<br><br>Conc. in ppm | Recovered viable cells cfu/ml | | | |
|---|---|---|---|---|
| | Sampling time after inoculation | | | |
| | 0 | 2 h | 6 h | 24 h |
| **Material D** | | | | |
| 500 ppm | $4.0 \times 10^4$ | 0 | 0 | 0 |
| 100 ppm | $1.1 \times 10^6$ | 0 | 0 | 0 |
| 20 ppm | $1.1 \times 10^6$ | 0 | 0 | 0 |
| 5 ppm | $1.0 \times 10^6$ | $9.2 \times 10^6$ | $1.6 \times 10^6$ | $4.1 \times 10^2$ |
| 1 ppm | $1.2 \times 10^6$ | $1.1 \times 10^6$ | $1.6 \times 10^6$ | $2.6 \times 10^6$ |
| **Kathon WT®** | | | | |
| 500 ppm | $9.5 \times 10^5$ | $2.2 \times 10^5$ | 5 | 0 |
| 100 ppm | $8.6 \times 10^5$ | $3.0 \times 10^5$ | $7.0 \times 10^2$ | 0 |
| 20 ppm | $8.7 \times 10^5$ | $2.2 \times 10^5$ | $1.2 \times 10^4$ | 0 |
| 5 ppm | $1.1 \times 10^6$ | $3.5 \times 10^5$ | $9.1 \times 10^3$ | 0 |
| 1 ppm | $9.1 \times 10^5$ | $8.0 \times 10^5$ | $4.9 \times 10^5$ | $6.0 \times 10^2$ |
| Control<br>(phosphate buffered<br>saline) | $1.2 \times 10^6$ | $1.3 \times 10^6$ | $1.8 \times 10^6$ | $2.3 \times 10^6$ |

10

## TABLE 7

Recovery of viable cells of Klebsiella pneumoniae NCTC 10341
following exposure to D and Kathon WT in
phosphate buffered saline

| Compound | Recovered viable cells cfu/ml | | | |
| --- | --- | --- | --- | --- |
| Conc. in ppm | Sampling time after inoculation | | | |
| | 0 | 2 h | 6 h | 24 h |
| **Material D** | | | | |
| 500 ppm | $6.8 \times 10^5$ | 0 | 0 | 0 |
| 200 ppm | $6.7 \times 10^5$ | 0 | 0 | 0 |
| 100 ppm | $7.9 \times 10^5$ | 0 | 0 | 0 |
| 50 ppm | $8.9 \times 10^5$ | 0 | 0 | 0 |
| 20 ppm | $7.0 \times 10^5$ | 9 | 0 | 0 |
| **Kathon WT** | | | | |
| 500 ppm | $8.4 \times 10^5$ | $8.2 \times 10^5$ | $1.5 \times 10^4$ | 0 |
| 200 ppm | $7.6 \times 10^5$ | $5.2 \times 10^5$ | $9.1 \times 10^4$ | $1.6 \times 10^5$ |
| 100 ppm | $8.3 \times 10^5$ | $6.1 \times 10^5$ | $2.8 \times 10^5$ | $3.9 \times 10^5$ |
| 50 ppm | $1.1 \times 10^6$ | $4.5 \times 10^5$ | $4.7 \times 10^5$ | $3.5 \times 10^4$ |
| 20 ppm | $1.0 \times 10^6$ | $4.6 \times 10^5$ | $8.4 \times 10^5$ | $3.1 \times 10^4$ |
| Control (phosphate buffered saline) | $9.2 \times 10^5$ | $7.8 \times 10^5$ | $9.5 \times 10^5$ | $2.2 \times 10^6$ |

## TABLE 8

Recovery of viable cells of Salmonella typhimurium NCIB 10258
following exposure to D and Kathon WT® in
phosphate buffered saline

| Compound Conc. in ppm | Recovered viable cells cfu/ml | | | |
|---|---|---|---|---|
| | Sampling time after inoculation | | | |
| | 0 | 2 h | 6 h | 24 h |
| **Material D** | | | | |
| 500 ppm | $9.0 \times 10^4$ | 0 | 0 | 0 |
| 100 ppm | $1.1 \times 10^6$ | 0 | 0 | 0 |
| 20 ppm | $1.0 \times 10^6$ | 0 | 0 | 0 |
| 5 ppm | $1.1 \times 10^6$ | $1.5 \times 10^6$ | $1.6 \times 10^6$ | $6.2 \times 10^6$ |
| 1 ppm | $1.0 \times 10^6$ | $1.3 \times 10^6$ | $1.6 \times 10^6$ | $8.1 \times 10^6$ |
| **Kathon WT®** | | | | |
| 500 ppm | $1.0 \times 10^6$ | $2.1 \times 10^5$ | $1.0 \times 10^4$ | 0 |
| 100 ppm | $1.3 \times 10^6$ | $9.7 \times 10^5$ | $1.1 \times 10^6$ | $3.6 \times 10^4$ |
| 20 ppm | $1.1 \times 10^6$ | $9.2 \times 10^5$ | $1.1 \times 10^6$ | $2.8 \times 10^5$ |
| 5 ppm | $1.2 \times 10^6$ | $1.1 \times 10^6$ | $1.2 \times 10^6$ | $4.3 \times 10^5$ |
| 1 ppm | $1.2 \times 10^6$ | $1.1 \times 10^6$ | $1.2 \times 10^6$ | $3.2 \times 10^5$ |
| Control (phosphate buffered saline) | $1.3 \times 10^6$ | $1.4 \times 10^6$ | $1.3 \times 10^6$ | $8.0 \times 10^6$ |

## TABLE 9

Recovery of viable cells of Salmonella enteritidis NCTC 6676
following exposure to D and Kathon WT in
phosphate buffered saline

| Compound | Recovered viable cells cfu/ml | | | |
|---|---|---|---|---|
| Conc. in ppm | Sampling time after inoculation | | | |
| | 0 | 2 h | 6 h | 24 h |
| Material D | | | | |
| 500 ppm | $3.4 \times 10^5$ | 0 | 0 | 0 |
| 200 ppm | $5.9 \times 10^5$ | 0 | 0 | 0 |
| 100 ppm | $7.7 \times 10^5$ | 0 | 0 | 0 |
| 50 ppm | $7.8 \times 10^5$ | 0 | 0 | 0 |
| 20 ppm | $8.5 \times 10^5$ | 16 | 0 | 0 |
| Kathon WT ® | | | | |
| 500 ppm | $6.1 \times 10^5$ | $3.1 \times 10^5$ | $2.1 \times 10^5$ | 0 |
| 200 ppm | $8.2 \times 10^5$ | $5.1 \times 10^5$ | $5.9 \times 10^5$ | $1.6 \times 10^3$ |
| 100 ppm | $5.0 \times 10^5$ | $6.0 \times 10^5$ | $6.0 \times 10^5$ | $1.5 \times 10^4$ |
| 50 ppm | $6.8 \times 10^5$ | $6.9 \times 10^5$ | $1.5 \times 10^5$ | $1.0 \times 10^4$ |
| 20 ppm | $7.6 \times 10^5$ | $6.1 \times 10^5$ | $3.1 \times 10^5$ | $3.3 \times 10^5$ |
| Control (phosphate buffered saline) | $7.9 \times 10^5$ | $7.5 \times 10^5$ | $1.0 \times 10^6$ | $3.3 \times 10^6$ |

## TABLE 10

Recovery of viable cells of Shigella boydii NCTC 9328
following exposure to D and Kathon® WT in
phosphate buffered saline

| Compound | Recovered viable cells cfu/ml | | | |
|---|---|---|---|---|
| Conc. in ppm | Sampling time after inoculation | | | |
| | 0 | 2 h | 6 h | 24 h |
| Material D | | | | |
| 500 ppm | $3.1 \times 10^5$ | 0 | 0 | 0 |
| 200 ppm | $3.9 \times 10^5$ | 0 | 0 | 0 |
| 100 ppm | $7.1 \times 10^5$ | 0 | 0 | 0 |
| 50 ppm | $6.6 \times 10^5$ | 0 | 0 | 0 |
| 20 ppm | $6.9 \times 10^5$ | $1.6 \times 10^2$ | 0 | 0 |
| Kathon WT® | | | | |
| 500 ppm | $5.2 \times 10^5$ | $1.6 \times 10^5$ | $6.0 \times 10^5$ | $1.5 \times 10^4$ |
| 200 ppm | $6.4 \times 10^5$ | $4.5 \times 10^5$ | $3.8 \times 10^5$ | $2.6 \times 10^3$ |
| 100 ppm | $6.8 \times 10^5$ | $6.2 \times 10^5$ | $4.4 \times 10^5$ | $1.8 \times 10^5$ |
| 50 ppm | $5.1 \times 10^5$ | $5.5 \times 10^5$ | $4.9 \times 10^5$ | $4.8 \times 10^5$ |
| 20 ppm | $5.9 \times 10^5$ | $6.8 \times 10^5$ | $6.3 \times 10^5$ | $7.4 \times 10^5$ |
| Control (phosphate buffered saline) | $6.9 \times 10^5$ | $9.8 \times 10^5$ | $1.3 \times 10^6$ | $3.0 \times 10^6$ |

## TABLE 11

Recovery of viable cells of Bacillus Subtilis ATCC 6633
following exposure to D and Kathon[(3)] WT in
phosphate buffered saline

| Compound<br><br>Conc. in ppm | Recovered viable cells cfu/ml | | | |
|---|---|---|---|---|
| | Sampling time after inoculation | | | |
| | 0 | 2 h | 6 h | 24 h |
| Material D | | | | |
| 500 ppm | $1.0 \times 10^3$ | $4.0 \times 10^2$ | $4.0 \times 10^2$ | 36 |
| 100 ppm | $1.2 \times 10^4$ | $9.0 \times 10^2$ | $7.0 \times 10^2$ | $2.5 \times 10^2$ |
| 20 ppm | $1.0 \times 10^4$ | $9.0 \times 10^2$ | $9.0 \times 10^2$ | $1.0 \times 10^3$ |
| 5 ppm | $1.0 \times 10^4$ | $7.0 \times 10^4$ | $4.9 \times 10^3$ | $1.1 \times 10^3$ |
| 1 ppm | $1.4 \times 10^5$ | $1.3 \times 10^5$ | $6.0 \times 10^4$ | $1.5 \times 10^5$ |
| Kathon WT[(2)] | | | | |
| 500 ppm | $6.0 \times 10^4$ | $1.0 \times 10^5$ | $2.0 \times 10^4$ | $1.9 \times 10^3$ |
| 100 ppm | $1.3 \times 10^5$ | $8.0 \times 10^4$ | $1.9 \times 10^4$ | $1.2 \times 10^3$ |
| 20 ppm | $1.1 \times 10^5$ | $9.0 \times 10^4$ | $2.0 \times 10^4$ | $2.4 \times 10^3$ |
| 5 ppm | $1.3 \times 10^5$ | $1.3 \times 10^5$ | $8.0 \times 10^4$ | $6.1 \times 10^3$ |
| 1 ppm | $1.3 \times 10^5$ | $1.0 \times 10^5$ | $1.3 \times 10^5$ | $9.9 \times 10^3$ |
| Control<br>(phosphate buffered<br>saline) | $1.6 \times 10^4$ | $9.8 \times 10^4$ | $1.6 \times 10^5$ | $2.0 \times 10^5$ |

## TABLE 12

Recovery of viable cells of Bacillus cereus ATCC 11778
following exposure to D and Kathon® WT in
phosphate buffered saline

| Compound | Recovered viable cells cfu/ml | | | |
|---|---|---|---|---|
| Conc. in ppm | Sampling time after inoculation | | | |
| | 0 | 2 h | 6 h | 24 h |
| Material D | | | | |
| 500 ppm | $1.4 \times 10^2$ | 10 | 6 | 1 |
| 100 ppm | $1.1 \times 10^5$ | 18 | 11 | 4 |
| 20 ppm | $3.3 \times 10^5$ | 33 | 12 | 12 |
| 5 ppm | $2.4 \times 10^5$ | $5 \times 10^4$ | $1.5 \times 10^2$ | 12 |
| 1 ppm | $2.7 \times 10^5$ | $1.8 \times 10^5$ | $1.4 \times 10^5$ | 9 |
| Kathon WT® | | | | |
| 500 ppm | $2.1 \times 10^5$ | $8.0 \times 10^4$ | $2.2 \times 10^5$ | 10 |
| 100 ppm | $2.3 \times 10^5$ | $1.1 \times 10^4$ | $1.1 \times 10^3$ | 16 |
| 20 ppm | $1.9 \times 10^5$ | $4.0 \times 10^3$ | $6.0 \times 10^2$ | 21 |
| 5 ppm | $2.3 \times 10^5$ | $5.0 \times 10^4$ | $5.0 \times 10^2$ | 51 |
| 1 ppm | $2.1 \times 10^5$ | $1.2 \times 10^5$ | $1.9 \times 10^3$ | 48 |
| Control (phosphate buffered saline) | $2.2 \times 10^5$ | $2.1 \times 10^5$ | $1.6 \times 10^5$ | $1.5 \times 10^5$ |

## TABLE 13

Recovery of viable cells of Candida albicans ATCC 10231
following exposure to D in phosphate buffered saline

| Compound | Recovered viable cells cfu/ml | | | |
| --- | --- | --- | --- | --- |
| Conc. in ppm | Sampling time after inoculation | | | |
| | 0 | 2 h | 6 h | 24 h |
| Material D | | | | |
| 500 ppm | $1.1 \times 10^5$ | $3.3 \times 10^4$ | $2.1 \times 10^3$ | 18 |
| 100 ppm | $9.5 \times 10^4$ | $7.5 \times 10^4$ | $7.5 \times 10^3$ | $1.2 \times 10^3$ |
| 20 ppm | $9.0 \times 10^4$ | $6.0 \times 10^4$ | $3.0 \times 10^3$ | $1.0 \times 10^4$ |
| 5 ppm | $6.0 \times 10^4$ | $6.1 \times 10^4$ | $2.0 \times 10^4$ | $4.0 \times 10^4$ |
| 1 ppm | $6.0 \times 10^4$ | $8.0 \times 10^4$ | $2.1 \times 10^4$ | $9.8 \times 10^4$ |
| Control (phosphate buffered saline) | $5.5 \times 10^4$ | $4.0 \times 10^4$ | $7.0 \times 10^4$ | $6.0 \times 10^4$ |

## TABLE 14

Recovery of viable cells of Candida tropicalis NCPF 3111
following exposure to D in phosphate buffered saline

| Compound | Recovered viable cells cfu/ml | | | |
| --- | --- | --- | --- | --- |
| Conc. in ppm | Sampling time after inoculation | | | |
| | 0 | 2 h | 6 h | 24 h |
| Material D | | | | |
| 500 ppm | $9.0 \times 10^4$ | $6.0 \times 10^4$ | $2.0 \times 10^4$ | 0 |
| 100 ppm | $5.9 \times 10^4$ | $5.0 \times 10^4$ | $5.0 \times 10^4$ | $9.1 \times 10^2$ |
| 20 ppm | $5.0 \times 10^4$ | $4.0 \times 10^4$ | $3.5 \times 10^3$ | $1.3 \times 10^4$ |
| 5 ppm | $1.4 \times 10^5$ | $4.3 \times 10^4$ | $4.1 \times 10^4$ | $1.0 \times 10^4$ |
| 1 ppm | $3.1 \times 10^4$ | $6.0 \times 10^4$ | $5.0 \times 10^4$ | $4.4 \times 10^4$ |
| Control (phosphate buffered saline) | $5.0 \times 10^4$ | $5.0 \times 10^4$ | $6.5 \times 10^4$ | $7.0 \times 10^4$ |

## TABLE 15

Recovery of viable cells of Absidia corymbifera NCPF 2001
following exposure to D in phosphate buffered saline

| Compound<br>Conc. in ppm | Recovered viable cells cfu/ml | | | |
|---|---|---|---|---|
| | Sampling time after inoculation | | | |
| | 0 | 2 h | 6 h | 24 h |
| Material D | | | | |
| 500 ppm | $1.9 \times 10^4$ | $1.0 \times 10^4$ | $2.0 \times 10^3$ | 0 |
| 100 ppm | $1.6 \times 10^4$ | $1.5 \times 10^4$ | $3.0 \times 10^3$ | $2.0 \times 10^3$ |
| 20 ppm | $1.7 \times 10^4$ | $1.5 \times 10^4$ | $1.6 \times 10^4$ | $1.4 \times 10^4$ |
| 5 ppm | $1.3 \times 10^4$ | $1.8 \times 10^4$ | $1.7 \times 10^4$ | $1.4 \times 10^4$ |
| 1 ppm | $1.5 \times 10^4$ | $1.6 \times 10^4$ | $1.7 \times 10^4$ | $6.0 \times 10^3$ |
| Control<br>(phosphate buffered<br>saline) | $1.8 \times 10^4$ | $1.4 \times 10^4$ | $1.7 \times 10^4$ | $1.6 \times 10^4$ |

## TABLE 16

Recovery of viable cells of Sporotrichum schenkii NCPF 3182
following exposure to D in  phosphate buffered saline

| Compound<br>Conc. in ppm | Recovered viable cells cfu/ml | | | |
|---|---|---|---|---|
| | Sampling time after inoculation | | | |
| | 0 | 2 h | 6 h | 24 h |
| Material D | | | | |
| 500 ppm | $4.0 \times 10^4$ | $9.6 \times 10^3$ | $1.8 \times 10^2$ | 0 |
| 100 ppm | $3.1 \times 10^4$ | $1.7 \times 10^4$ | $1.0 \times 10^4$ | $6.1 \times 10^3$ |
| 20 ppm | $2.6 \times 10^4$ | $3.0 \times 10^4$ | $3.4 \times 10^4$ | $2.2 \times 10^4$ |
| 5 ppm | $2.7 \times 10^4$ | $3.5 \times 10^4$ | $4.0 \times 10^4$ | $4.0 \times 10^4$ |
| 1 ppm | $3.4 \times 10^4$ | $2.7 \times 10^4$ | $3.6 \times 10^4$ | $4.1 \times 10^4$ |
| Control<br>(phosphate buffered<br>saline) | $2.9 \times 10^4$ | $3.3 \times 10^4$ | $3.0 \times 10^4$ | $6.0 \times 10^4$ |

## TABLE 17

Recovery of viable cells of Trichophyton rubrum NCPF 197
following exposure to D in phosphate buffered saline

| Compound | Recovered viable cells cfu/ml | | | |
|---|---|---|---|---|
| Conc. in ppm | Sampling time after inoculation | | | |
| | 0 | 2 h | 6 h | 24 h |
| Material D | | | | |
| 500 ppm | $2.0 \times 10^3$ | 0 | 0 | 0 |
| 100 ppm | $3.0 \times 10^3$ | $3.0 \times 10^3$ | 10 | 0 |
| 20 ppm | $4.0 \times 10^3$ | $2.0 \times 10^3$ | $9.0 \times 10^2$ | $3.4 \times 10^2$ |
| 5 ppm | $3.0 \times 10^3$ | $3.0 \times 10^3$ | $3.0 \times 10^2$ | $2.0 \times 10^3$ |
| 1 ppm | $2.0 \times 10^3$ | $2.5 \times 10^3$ | $5.0 \times 10^3$ | $3.0 \times 10^3$ |
| Control (phosphate buffered saline) | $6.0 \times 10^3$ | $4.0 \times 10^3$ | $3.0 \times 10^3$ | $9.0 \times 10^4$ |

## TABLE 18

Recovery of viable cells of Trichophyton mentagrophytes NCPF 410
following exposure to D in phosphate buffered saline

| Compound | Recovered viable cells cfu/ml | | | |
|---|---|---|---|---|
| Conc. in ppm | Sampling time after inoculation | | | |
| | 0 | 2 h | 6 h | 24 h |
| Material D | | | | |
| 500 ppm | $1.6 \times 10^4$ | 0 | 0 | 0 |
| 100 ppm | $2.1 \times 10^4$ | $1.2 \times 10^4$ | $1.7 \times 10^2$ | 0 |
| 20 ppm | $1.7 \times 10^4$ | $2.1 \times 10^4$ | $1.4 \times 10^4$ | $4.0 \times 10^2$ |
| 5 ppm | $1.5 \times 10^4$ | $2.6 \times 10^4$ | $1.9 \times 10^4$ | $3.0 \times 10^3$ |
| 1 ppm | $1.8 \times 10^4$ | $1.9 \times 10^4$ | $2.6 \times 10^4$ | $6.0 \times 10^3$ |
| Control (phosphate buffered saline) | $1.8 \times 10^4$ | $2.0 \times 10^4$ | $1.9 \times 10^4$ | $4.5 \times 10^5$ |

EP 0 275 207 B1

## TABLE 19

Recovery of viable cells of Microsporum canis NCPF 351
following exposure to D in phosphate buffered saline

| Compound<br><br>Conc. in ppm | Recovered viable cells cfu/ml | | | |
|---|---|---|---|---|
| | Sampling time after inoculation | | | |
| | 0 | 2 h | 6 h | 24 h |
| Material D<br><br>500 ppm<br>100 ppm<br>20 ppm<br>5 ppm<br>1 ppm | $1.3 \times 10^4$<br>$1.5 \times 10^4$<br>$1.2 \times 10^4$<br>$1.3 \times 10^4$<br>$1.0 \times 10^4$ | $3.8 \times 10^2$<br>$8.0 \times 10^3$<br>$1.4 \times 10^4$<br>$8.0 \times 10^3$<br>$1.1 \times 10^4$ | $10$<br>$3.0 \times 10^3$<br>$6.0 \times 10^3$<br>$6.0 \times 10^3$<br>$1.1 \times 10^4$ | $0$<br>$30$<br>$4.2 \times 10^2$<br>$7.0 \times 10^3$<br>$1.0 \times 10^4$ |
| Control<br>(phosphate buffered<br>saline) | $1.0 \times 10^4$ | $1.1 \times 10^4$ | $1.2 \times 10^4$ | $9.0 \times 10^3$ |

## TABLE 20

Recovery of viable cells of Penicillium funiculosum CMI 114933
following exposure to D in phosphate buffered saline

| Compound<br><br>Conc. in ppm | Recovered viable cells cfu/ml | | | |
|---|---|---|---|---|
| | Sampling time after inoculation | | | |
| | 0 | 2 h | 6 h | 24 h |
| Material D<br><br>500 ppm<br>100 ppm<br>20 ppm<br>5 ppm<br>1 ppm | $8.0 \times 10^3$<br>$8.0 \times 10^3$<br>$1.1 \times 10^4$<br>$9.0 \times 10^3$<br>$1.1 \times 10^4$ | $6.0 \times 10^3$<br>$6.0 \times 10^3$<br>$4.0 \times 10^3$<br>$7.0 \times 10^3$<br>$7.0 \times 10^3$ | $6.0 \times 10^3$<br>$9.0 \times 10^3$<br>$8.0 \times 10^3$<br>$7.0 \times 10^3$<br>$7.0 \times 10^3$ | $10$<br>$8.0 \times 10^3$<br>$5.0 \times 10^3$<br>$7.0 \times 10^3$<br>$6.0 \times 10^3$ |
| Control<br>(phosphate buffered<br>saline) | $1.4 \times 10^4$ | $1.0 \times 10^4$ | $9.0 \times 10^3$ | $1.0 \times 10^4$ |

20

## TABLE 21

Recovery of viable cells of Aureobasidium pullulans CMI 45533
following exposure to D in phosphate buffered saline

| Compound | Recovered viable cells cfu/ml | | | |
|---|---|---|---|---|
| Conc. in ppm | Sampling time after inoculation | | | |
| | 0 | 2 h | 6 h | 24 h |
| Material D | | | | |
| 500 ppm | $4.0 \times 10^3$ | $1.9 \times 10^3$ | $2.0 \times 10^3$ | $2.0 \times 10^3$ |
| 100 ppm | $2.8 \times 10^3$ | $2.1 \times 10^3$ | $3.1 \times 10^3$ | $2.5 \times 10^3$ |
| 20 ppm | $3.0 \times 10^3$ | $3.0 \times 10^3$ | $3.0 \times 10^3$ | $1.9 \times 10^3$ |
| 5 ppm | $3.0 \times 10^3$ | $1.9 \times 10^3$ | $2.1 \times 10^3$ | $3.6 \times 10^3$ |
| 1 ppm | $4.1 \times 10^3$ | $2.0 \times 10^3$ | $2.3 \times 10^3$ | $2.2 \times 10^3$ |
| Control (phosphate buffered saline) | $3.0 \times 10^3$ | $2.6 \times 10^3$ | $2.6 \times 10^3$ | $3.0 \times 10^4$ |

Example 2

The activity of 50% aqueous bis THP phosphite, 50% aqueous mono THP phosphite and 80% aqueous THP sulphate against Trichophyton rubrum were compared in a 7 day test in phosphate buffered saline solution to determine $ED_{50}$ and $ED_{90}$ values.

<u>Table 22</u>

| Concentration ppm (Active Compound) | 50% bis THP phosphite Mg. growth | | 50% THP phosphite Mg. growth | | 80% THP Sulphate Mg. growth | |
|---|---|---|---|---|---|---|
| | dry weight | (% of control) | dry weight | (% of control) | dry weight | (% of control) |
| 0 | 10.6 | (100) | 7.1 | (100) | 8 | (100) |
| 5 | 8.7 | (82) | 9.3 | (131) | - | - |
| 8 | - | - | - | - | 7.3 | (92) |
| 15 | 2.1 | (20) | 2.8 | (39) | - | - |
| 24 | - | - | - | - | 0 | (0) |
| 50 | 0 | (0) | 3.1 | (44) | - | - |
| 80 | - | - | - | - | 0 | (0) |
| 150 | 0 | (0) | 2.3 | (32) | - | - |
| 240 | - | - | - | - | 0 | (0) |
| 500 | 0 | (0) | 1.6 | (23) | - | - |
| 800 | - | - | - | - | 0 | (0) |
| $ED_{50}$ | 10 ppm | | 11 ppm | | 14 ppm | |
| $ED_{90}$ | 25 ppm | | >500 ppm | | 20 ppm | |

THP chloride, bis THP phosphate, THP borate, THP bromide, THP fluoride, THP acetate, THP citrate, THP lactate and THP tartrate all give substantially similar $ED_{50}$ and $ED_{90}$ results against T. Rubrum to those obtained using bis THP phosphite and THP sulphate.

In contrast under the same conditions phosphorous acid, which is widely used as a fungicide, requires concentrations of the order of 0.1 to 0.2% by weight to produce a useful reduction in the growth of T. Rubrum.

**Example 3**

THPS was found to give a complete in vitro kill of Naegleria (the protozoon responsible for Amoebic Meningitis) at concentrations of 15 ppm.

**Example 4**

One of ten Koi Carp, in a fish pond, exhibited symptoms of protozoal infection. 100 ppm of 75% aqueous THP sulphate was dosed to the pond via the oxygenating venturi to provide a concentration of 27 ppm in the pond water. The infected fish recovered fully and none of the other fish became infected. The fish fed normally throughout the treatment and exhibited no signs of distress.

**Example 5**

A cream for treatment of fungal infections of human skin by topical application comprises:
9% emulsifying wax
15% soft white parafin
6% liquid parafin
0.5% citric acid monohydrate
2.5% sodium phosphate
2% THP phosphate
balance sterile water
When the cream is applied twice daily to areas of ringworm infection full remission is normally observed within 1 to 2 weeks.

**Example 6**

A mouthwash for dilution with an equal volume of warm water prior to use comprises:
1.5gm sodium chloride
1.0gm sodium bicarbonate
2.5ml peppermint emulsion
1ml 75% THP chloride solution
50ml chloroform water
balance to 100ml sterile water
The composition produces rapid alleviation of oral thrush.

**Example 7**

Nasal drops comprise:
500 mg Ephedrine hydrochloride
500 mg Chlorobutanol
500 mg Sodium Chloride
1ml 75% aqueous THP chloride
balance to 100ml water

**Example 8**

Foot powder comprises:
25% zinc oxide
25% starch
50% talc
The starch is first mixted with 12%, by weight thereof of 75% aqueous THP sulphate.
The powder produces rapid alleviation of Athletes Foot.

EP 0 275 207 B1

**Example 9**

A foot spray comprises the starch preparation of example 8 dispersed in a pressure liquified propellent gas, together with a perfume and an antiperspirant. The composition provides reduction in foot odours and is useful in preventing or alleviating Athletes Foot

**Claims**

1. A therapeutic or prophylactic composition comprising a hydroxyalkylphosphine compound of the formula $(HORPR^1{}_n)_xX_y$ wherein R is an alkylene group having from 1 to 4 carbon atoms and each $R^1$ may be the same or different and is an alkyl group having from 1 to 4 carbon atoms or an ROH group, X is an anion such that the compound is at least sparingly soluble in water, n is 2 or 3 and y is 0 or 1 such that $(n+y)$ is 2 or 4; or an at least sparingly water soluble condensate thereof; and a pharmaceutically or veterinarily acceptable carrier or diluent.

2. Composition according to claim 1 wherein R has 1 carbon atom.

3. Composition according to either of claims 1 and 2 wherein each $R^1$ has 1 carbon atom.

4. Composition according to any foregoing claim wherein $R^1$ is R(OH).

5. Composition according to claim 1 wherein the hydroxyalkylphosphine compound is a water soluble tetrakis (hydroxymethyl) phosphonium salt.

6. Composition according to any of claims 1 to 5 which is antiseptic and/or antifungal cream, lotion, paste, collodion or ointment for topical application to the human or animal body.

7. An ointment according to claim 6 comprising liquid paraffin and/or soft paraffin and/or wool fat and/or wool fat derivative.

8. A cream according to claim 6 comprising the composition according to claim 7 dispersed or emulsified in water.

9. Composition according to any of claims 1 to 5 which is a powder for application to the human or animal body.

10. A composition according to claim 9 comprising talc, zinc oxide, and/or starch or a derivative thereof.

11. Composition according to any of claims 1 to 5 which is a pessary.

12. Composition according to any of claims 1 to 5 which is an aerosol spray composition.

13. Composition according to any of claims 1 to 5 which is a soluble capsule.

14. Composition according to any one of claims 1 to 5 which is a tablet.

15. Composition according to any one of claims 1 to 5 which is an aqueous composition for oral or parenteral administration.

16. A mouthwash according to claim 15.

17. Nasal drops according to claim 15.

18. Composition according to any one of claims 1 to 5 which is a soap bar containing at least 0.2% by weight of said hydroxyalkylphosphine compound.

19. Composition according to any one of claims 1 to 5 which is a veterinary dip containing at least 0.2% by weight of said hydroxyalkylphosphine compound.

24

**20.** A composition according to any of claims 1 to 19 containing from 0.2% to 20% by weight of said hydroxyalkylphosphine compound.

**21.** A composition according to claim 20 containing from 1 to 10% by weight of said hydroxyalkyl phosphine compound.

**22.** A composition according to any of claims 1 to 21 additionally comprising at least one other active ingredient selected from fungicides, bactericides, synergists, antiperspirants, styptics, carboxymethyl cellulose, soaps, surfactants, polymeric thickening agents, wetting agents, foam controlling agents, perfumes, flavouring, colouring, deodorants, reodorants and antiseptics.

**23.** A concentrate adapted on dilution with water to provide a composition according to any of claims 1 to 22.

**Revendications**

**1.** Composition thérapeutique ou prophylactique, comprenant un composé d'hydroxyalkylphosphine de formule $(HORPR^1n)_xX_y$, où R est un groupe alkylène ayant de 1 à 4 atomes de carbone et où chaque $R^1$ peut être identique ou différent et est un groupe alkyle ayant de 1 à 4 atomes de carbone ou un groupe ROH, X est un anion tel que le composé est au moins difficilement soluble dans l'eau, n est égal à 2 ou 3 et y est égal à 0 ou 1, de façon à ce que $(n+y)$ soit égal à 2 ou 4; ou un produit de condensation de celui-ci au moins difficilement soluble dans l'eau et un support ou diluant acceptable d'un point de vue pharmaceutique ou vétérinaire.

**2.** Composition selon la revendication 1 dans laquelle R a un atome de carbone.

**3.** Composition selon l'une quelconque des revendications 1 et 2, dans laquelle chaque $R^1$ a un atome de carbone.

**4.** Composition selon l'une quelconque des revendications précédentes, dans laquelle $R^1$ est R(OH).

**5.** Composition selon la revendication 1, dans laquelle le composé d'hydroxyalkylphosphine est un sel soluble dans l'eau de tétrakis-hydroxyméthylphosphonium.

**6.** Composition selon l'une quelconque des revendications 1 à 5, qui est une crème, une lotion, une pâte, un collodion ou un onguent antiseptique et/ou fongicide pour application topique sur les corps humains ou animaux.

**7.** Onguent selon la revendication 6, comprenant de la paraffine liquide et/ou de la paraffine molle et/ou de la lanoline et/ou un dérivé de la lanoline.

**8.** Crème selon la revendication 6, comprenant la composition selon la revendication 7, dispersée ou émulsifiée dans l'eau.

**9.** Composition selon l'une quelconque des revendications 1 à 5, qui est une poudre pour application aux corps humains ou animaux.

**10.** Composition selon la revendication 9, comprenant du talc, de l'oxyde de zinc et/ou de l'amidon ou un dérivé de celui-ci.

**11.** Composition selon l'une quelconque des revendications 1 à 5, qui est un pessaire.

**12.** Composition selon l'une quelconque des revendications 1 à 5, qui est une composition de pulvérisation en aérosol.

**13.** Composition selon l'une quelconque des revendications 1 à 5, qui est une capsule soluble.

**14.** Composition selon l'une quelconque des revendications 1 à 5, qui est une pastille.

**15.** Composition selon l'une quelconque des revendications 1 à 5, qui est une composition aqueuse pour administration orale ou parentérale.

**16.** Bain de bouche selon la revendication 15.

**17.** Gouttes nasales selon la revendication 15.

**18.** Composition selon l'une quelconque des revendications 1 à 5, qui est un pain de savon contenant au moins 0,2% en poids dudit composé d'hydroxyalkylphosphine.

**19.** Composition selon l'une quelconque des revendications 1 à 5, qui est un bain vétérinaire contenant au moins O, 2% en poids dudit composé d'hydroxyalkylphosphine.

**20.** Composition selon l'une quelconque des revendications 1 à 19, contenant de 0,2% à 20% en poids dudit composé d'hydroxyalkylphosphine.

**21.** Composition selon la revendication 20, contenant de 1 à 10% en poids dudit composé d'hydroxyalkylphosphine.

**22.** Composition selon l'une quelconque des revendications 1 à 21, comprenant en outre au moins un autre ingrédient actif choisi parmi les fongicides, les bactéricides, les agents synergiques, les antitranspi-rants, les styptiques, la carboxyméthyl-cellulose, les savons, les agents tensio-actifs, les agents d'épaississement polymères, les agents mouillants, les agents contrôlant les mousses, les parfums, les aromates, les colorants, les déodorants, les réodorants et les antiseptiques.

**23.** Concentré adapté pour la dilution avec de l'eau pour obtenir une composition selon l'une quelconque des revendications 1 à 22.

**Patentansprüche**

**1.** Therapeutische oder prophylaktische Zusammensetzung, enthaltend eine Hydroxyalkylphosphinverbin-dung der Formel $(HORPR^1_n)_xX_y$, in welcher R für eine Alkylengruppe mit 1 bis 4 Kohlenstofftomen steht, jedes $R^1$ gleich oder verschieden sein kann und eine Alkylgruppe mit 1 bis 4 Kohlenstoffatomen oder eine ROH-Gruppe bedeutet, X ein Anion darstellt, sodaß die Verbindung zumindest geringfügig in Wasser löslich ist, n für 2 oder 3 und y für 0 oder 1 steht, sodaß (n + y) 2 oder 4 bedeutet; oder ein zumindest geringfügig wasserlösliches Kondensat derselben; und einen pharmazeutisch oder veterinär verwendbaren Träger oder ein Verdünnungsmittel.

**2.** Zusammensetzung nach Anspruch 1, in welcher R 1 Kohlenstoffatom enthält.

**3.** Zusammensetzung nach Anspruch 1 oder 2, in welcher jedes $R^1$ 1 Kohlenstoffatom enthält.

**4.** Zusammensetzung nach einem der vorhergehenden Ansprüche, in welcher $R^1$ für R(OH) steht.

**5.** Zusammensetzung nach Anspruch 1, in welcher die Hydroxyalkylphosphinverbindung ein wasserlösliches Tetrakis(hydroxymethyl)-phosphoniumsalz ist.

**6.** Zusammensetzung nach einem der Ansprüche 1 bis 5, die eine antiseptische und/oder antifungale Creme, Lotion, Paste, ein Kollodium oder eine Salbe zur topischen Anwendung für den menschlichen oder tierischen Körper ist.

**7.** Salbe nach Anspruch 6, enthaltend flüssiges Paraffin und/oder Weichparaffin und/oder Wollfett und/oder ein Wollfettderivat.

**8.** Creme nach Anspruch 6, enthaltend die Zusammensetzung nach Anspruch 7, dispergiert oder emulgiert in Wasser.

**9.** Zusammensetzung nach einem der Ansprüche 1 bis 5, die ein Puder zur Anwendung für den menschlichen oder tierischen Körper ist.

**10.** Zusammensetzung nach Anspruch 9, enthaltend Talkum, Zinkoxid und/oder Stärke oder ein Derivat derselben.

**11.** Zusammensetzung nach einem der Ansprüche 1 bis 5, die ein Pessar ist.

**12.** Zusammensetzung nach einem der Ansprüche 1 bis 5, die eine Aerosol-Sprayzusammensetzung ist.

**13.** Zusammensetzung nach einem der Ansprüche 1 bis 5, die eine lösliche Kapsel ist.

**14.** Zusammensetzung nach einem der Ansprüche 1 bis 5, die eine Tablette ist.

**15.** Zusammensetzung nach einem der Ansprüche 1 bis 5, die eine wässerige Zusammensetzung zur oralen oder parenteralen Verabreichung ist.

**16.** Mundspülung nach Anspruch 15.

**17.** Nasentropfen nach Anspruch 15.

**18.** Zusammensetzung nach einem der Ansprüche 1 bis 5, die ein Seifenriegel ist, der zumindest 0,2 Gew.-% der genannten Hydroxyalkylphosphinverbindung enthält.

**19.** Zusammensetzung nach einem der Ansprüche 1 bis 5, die ein veterinär anwendbares Tauchbad ist, das zumindest 0,2 Gew.-% der genannten Hydroxyalkylphosphinverbindung enthält.

**20.** Zusammensetzung nach einem der Ansprüche 1 bis 19, die 0,2 Gew.-% bis 20 Gew.-% der genannten Hydroxyalkylphosphinverbindung enthält.

**21.** Zusammensetzung nach Anspruch 20, die 1 Gew.-% bis 10 Gew.-% der genannten Hydroxyalkylphosphinverbindung enthält.

**22.** Zusammensetzung nach einem der Ansprüche 1 bis 21, die zusätzlich zumindest einen weiteren Wirkstoff, ausgewählt aus Fungiziden, Bakteriziden, Synergistika, Antiperspirantien, blutstillenden Mitteln, Carboxymethylcellulose, Seifen, oberflächenaktiven Mitteln, polymeren Verdickungsmitteln, Netzmitteln, schaumkontrollierenden Mitteln, Duftstoffen, Geschmackstoffen, Farbstoffen, Desodorantien, Reodorantien und Antiseptika enthält.

**23.** Konzentrat zur Verdünnung mit Wasser, um eine Zusammensetzung nach einem der Ansprüche 1 bis 22 zu ergeben.